Europäisches Patentamt

European Patent Office

Office européen des brevets

(11) Publication number: **0 053 657**
**B1**

## (12) EUROPEAN PATENT SPECIFICATION

(45) Date of publication of patent specification: **24.04.85**

(21) Application number: **81107076.2**

(22) Date of filing: **09.09.81**

(51) Int. Cl.⁴: **C 07 C 21/18, C 07 C 17/00**

(54) **Preparation of chlorotrifluoroethylene and trifluoroethylene.**

(30) Priority: **09.12.80 US 214550**

(43) Date of publication of application:
**16.06.82 Bulletin 82/24**

(45) Publication of the grant of the patent:
**24.04.85 Bulletin 85/17**

(84) Designated Contracting States:
**DE FR GB IT**

(56) References cited:
**GB-A- 698 386**
**US-A-2 697 124**
**US-A-2 864 873**
**US-A-3 333 011**
**US-A-4 226 812**

(73) Proprietor: **ALLIED CORPORATION**
**Columbia Road and Park Avenue P.O. Box 2245R**
**(Law Dept.)**
**Morristown New Jersey 07960 (US)**

(72) Inventor: **Cunningham, William Joseph**
**172 Country-side Lane**
**Williamsville New York 14221 (US)**
Inventor: **Piskorz, Ronald Fabian**
**15 Kaufman Road**
**Cheektowaga New York 14226 (US)**
Inventor: **Smith, Addison Miles**
**80 Berryman Drive**
**Buffalo New York 14226 (US)**

(74) Representative: **Madgwick, Paul Roland et al**
**Ladas & Parry Isartorplatz 5**
**D-8000 München 2 (DE)**

Courier Press, Leamington Spa, England.

## Description

### Background of the invention

Tetrafluoroethane, and particularly asymmetrical tetrafluoroethane, has been suggested for refrigeration and other applications where chlorofluorocarbons and other fluorocarbons are now used. German published patent application DOS 2,822,471 describes the production of tetrafluoroethane by hydrogenation of chlorofluoroethanes such as symmetrical and asymmetrical dichlorotetrafluoroethane. U.S. Patent 4,129,603 discloses the manufacture of asymmetrical tetrafluoroethane by reacting 1,1,1-trifluoro-2-chloroethane with hydrogen fluoride in the presence of a chromium oxide catalyst and removing certain unsaturated impurities by contacting the crude product with aqueous permanganate salts.

It has been found that both of these methods for producing tetrafluoroethane have in the crude products materials such as asymmetrical dichlorotetrafluoroethane (fluorocarbon 114a) and asymmetrical trifluoroethane (fluorocarbon 143a). Both of these materials are now believed to form azeotropes with tetrafluoroethane and especially asymmetrical tetrafluoroethane. Under these conditions, it is difficult to separate tetrafluoroethane from such impurities by standard distillation techniques.

The production of chlorotrifluoroethylene (fluorocarbon 1113) and trifluoroethylene (fluorocarbon 1123) from trichlorotrifluoroethane is described, for example, in U.S. Patent 2,802,887 to *Miller* et al. (August 13, 1957) and U.S. Patent 3,564,064 to *Nakagawa* (February 16, 1971). Both of these patents employ a palladium or platinum on an activated carbon or alumina catalyst. Similar processes limited to production of chlorotrifluoroethylene from trichlorotrifluoroethane, (fluorocarbon 113) but with other catalysts, are known from references such as U.S. Patents 2,704,775 to *Clark* (March 22, 1955) and 2,697,124 to *Mantell* (December 14, 1954). While the Mantell patent discloses magnesium among a broad category of catalyst supports, the specificity of the recyclability of the catalyst when sodium or potassium magnesium fluoride support is used is not recognized.

Chlorotrifluoroethylene is useful as a monomer in fluoropolymers, while trifluoroethylene can be hydrofluorinated to tetrafluoroethane. Tetrafluoroethane produced from trifluoroethylene is easier to purify by distillation than tetrafluoroethane produced by other techniques.

### Brief description of the invention

The present invention includes a process for producing chlorotrifluoroethylene or trifluoroethylene by the dechlorination in the vapor phase of 1,1,2-trichloro-1,2,2-trifluoroethane or chlorotrifluoroethylene with hydrogen in the presence of a supported platinum-group metal catalyst, wherein a feed of 1,1,2-trichloro-1,2,2-trifluoroethane, chlorotrifluoroethylene or mixtures thereof are reacted with hydrogen at a reaction temperature of 175 to 300°C in the presence of a supported platinum-group metal catalyst, characterized in that the catalyst support is selected from the group consisting of sodium magnesium fluoride and potassium magnesium fluoride, the catalyst is contacted with an oxygen-containing gas at a temperature between 400 and 600°C to reactivate the catalyst, and the feed is reacted with hydrogen in the presence of the reactivated catalyst at the reaction temperature.

The present invention also includes a process for producing chlorotrifluoroethylene by the catalytic dechlorination in the vapor phase of 1,1,2-trichloro-1,2,2-trifluoroethane with hydrogen at a temperature of between 400 and 600°C in the presence of a catalyst selected from the group consisting of sodium magnesium fljoride and potassium magnesium fluoride that may be coated with at least one of iron, copper and nickel and periodically reactivating the catalyst by contacting the catalyst with an oxygen-containing gas at a temperature between about 400°C and about 600°C.

### Detailed description of the invention

The present invention relates particularly to a vapor phase dechlorination reaction in the presence of a catalyst. The catalyst for the lower-temperature reactor is a supported platinum-group metal, i.e. ruthenium, rhodium, palladium, osmium, iridium or platinum and is preferably palladium. Under the reducing conditions of the reaction, the metal is partly or even predominantly at a zero valence state, but may include salts or oxides with the platinum-group metal at a positive valence state.

The support is an alkali magnesium fluoride such as sodium or potassium magnesium fluoride. It may be formed by precipitating magnesium and alkali metals with fluoride from aqueous solutions of water-soluble alkali metal and magnesium salts such as the chlorides. The precipitation is designed to form a mixed salt wherein the alkali metal and magnesium are in substantially equimolar amounts as described in *Zeitschrift fur Anorganische and Allgemeine Chemie*, bond 355-280-1967. The support may contain some chloride in place of fluoride, derived either from the aqueous solution used to make support or by halogen replacement with HCl under dechlorination reaction conditions. The support may also contain other cations or alteration in the proportion of cations without significant loss of activity.

Catalyst particles for the low-temperature reaction may be conveniently prepared by drying, grinding and screening the precipitated alkali magnesium fluoride and then coating the resultant particles with a palladium-metal salt such as $PdCl_2$. The coated particles may then be dried and the palladium metal at least partly reduced to a zero valence state with hydrogen, conveniently at the reaction temperature.

For the higher temperature reaction, the alkali magnesium fluoride may be used uncoated or coated with at least one of iron, copper or nickel. The preferred catalysts are the uncoated particles and those coated with nickel. Sodium magnesium fluoride is preferred, with potassium magnesium fluoride also being satisfactory. If the particles are to be used as such, one should directly activate the screened particles. If copper, iron or nickel are to be coated on, they should be coated on as a water soluble salt, then dried, and then optionally activated with hydrogen alone before introducing fluorocarbon 113.

During the dechlorination reaction various deposits build up on the catalyst which gradually reduce its activity. This build-up, apparently in the form of polymeric material and a coke, also occurs with other supports for palladium such as activated carbon. Unlike the palladium-on-activated carbon supports of the prior art, however, the present catalysts can be reactivated by exposure at elevated temperatures, such as about 400°C to about 600°C, preferably about 500—550°C, to an oxygencontaining gas such as air. It is preferred to contact the reactivated catalyst with hydrogen only at the reaction temperature before resuming the feed of chlorofluorocarbon.

The feed may be 1,1,2-trichloro-1,2,2-trifluoroethane (fluorocarbon 113) used to produce chlorotrifluoroethylene (fluorocarbon 1113) or trifluoroethylene (fluorocarbon 1123) based on reaction with two or three molecules of hydrogen with each molecule of fluorocarbon 113. Alternatively, the feed may be fluorocarbon 1113 (which is commercially available) used to produce fluorocarbon 1123 by reaction of one molecule of hydrogen with each molecule of fluorocarbon 1113. In the high temperature reaction, only the feed of fluorocarbon 113 to produce fluorocarbon 1113 is feasible.

It is preferred that mixtures of fluorocarbons 113 and 1113 be fed in the low temperature reaction to produce fluorocarbon 1123. It has been found that the dechlorination of fluorocarbon 113 to fluorocarbon 1123 is enhanced by the presence of fluorocarbon 1113 (the intermediate) in the feed. This can be accomplished by recycling fluorocarbon 1113 from the effluent of the reaction and using fluorocarbon 113 as the feed since, with hydrogen being provided at less than large excesses, some of the fluorocarbon 113 will be dechlorinated only to fluorocarbon 1113. By contrast, Canadian Patent 655,897 claims to minimize the production of fluorocarbon 1113 when dechlorinating fluorocarbon 113 to fluorocarbon 1123. Fluorocarbon 1113 is acceptable or even desirable in the effluent if it is to be recycled. By contrast, 1,1,2-trifluoroethane (fluorocarbon 143) which may be formed by the addition of a fourth mole of hydrogen to fluorocarbon 1123 is considered an undesired by-product that can be reclaimed only by chlorinating the entire distance back to fluorocarbon 113. If fluorocarbons 133 and 123a are formed during the reaction, they can be recycled.

The level of palladium-group metal does not appear especially critical, with the range of not more than 2.5% of total weight of catalyst (preferably 0.5—2.0%) stated for palladium-on-activated carbon in Canadian Patent 655,897 being satisfactory. Similar levels of other metals are used in the higher temperature catalyst. Contact times are also not critical, with a range of 1 to 10 seconds being satisfactory.

The reaction temperature when using a platinum group metal coated catalyst is about 175—300°C, preferably about 200—250°C. The ratio of hydrogen to chlorofluorocarbon fed may vary from as little as 0.1 to 1 to as much as 2 to 1 or even higher. In general more hydrogen should be fed as the proportion of fluorocarbon 113 in the feed increases since fluorocarbon 113 has three chlorines to remove rather than one. When fluorocarbon 1113 is the sole feed, the above ratio should not exceed 1 to 1. The hydrogen level is too high when fluorocarbon 143 can be detected in significant quantities in the effluent.

When fluorocarbon 1123 is produced by the present process, it may be hydrofluorinated to produce assymmetrical tetrafluoroethane. A catalyst vapor phase process is described in Belgian Patent 870,530 of Imperial Chemicals Industries, Ltd. (March 15, 1979).

The reaction temperature for uncoated, iron coated, nickel coated or copper coated catalysts is about 400 to 600°C, preferably about 475°C to about 550°C. The feed ratio is preferably below about 1:1 hydrogen:fluorocarbon 113 since higher levels of hydrogen increase the deactivation rate of the catalyst and thus requires more frequent reactivation.

Example 1

Preparation of $NaMgF_3$ and $NaMgF_3$—supported catalysts

317 g (7.55 mol) sodium fluoride (NaF) was dissolved in 7 L of distilled water in a 12 L glass flask. The mixture was heated to 90—95°C. 487.3 g (3.00 mol) of magnesium chloride ($MgCl_2 . 6H_2O$) was separately dissolved in 1 L of distilled water and this solution added to the sodium fluoride solution dropwise over a period of about 1.5 h with mechanical agitation. Agitation and temperature (90—95°C) were maintained for 1 h more. The mixture was allowed to cool and settle overnight. The clear liquid (aqueous NaCl) was siphoned off and the precipitate vacuum filtered through #4 Whatman paper. While the cake was still wet it was washed with 10—100 mL portions of water and then filtered semidry. The cake was dried overnight at 160°C in a vacuum oven.

When used "neat" (without impregnating a metal) this dried cake was calcined at 550°C in a muffled furnace for 16 hours, broken into chunks and screened for particles between American standard mesh 8 (about 2.0 mm) and American Standard mesh 20 (about 0.71 mm).

For the coated catalyst, the filter cake was calcined at 550°C in a muffled furnace for 16 hours, broken into chunks and screened for particles between American Standard mesh 8 (2.0 mm diameter) and American Standard mesh 20 (0.71 mm diameter). Palladium was then added as an aqueous $PdCl_2$ solution (with HCl as required to completely dissolve the $PdCl_2$). The wet coated catalyst was dried in a steam bath

3

until free flowing. The resultant granules were then dried overnight at 160°C in a vacuum oven. The dried catalyst was then placed in the reactor tube (described below) and treated with hydrogen for about 2 hours at 200°C to convert the $PdCl_2$ to elemental palladium.

Example 2
Dechlorination of fluorocarbon 113 with $NaMgF_3$ supported catalysts

A 1 inch by 30 inch (2.54 by 76.2 cm) stainless steel pipe reactor lined with a 3/4 inch (1.91 cm) inside diameter Coors 998 pure fused alumina liner was fitted with a 1/4 inch (0.64 cm) diameter Coors alumina thermowel running its entire length from the gas feed at the bottom to the product exit at the top for gases flowing between the thermowell and the liner. The reactor was placed in a vertically mounted 24 inch, three zone, electrically heated tube furnace. The effluent line was fitted with for on-line gas chromatography analysis and acid determination in water by caustic titration.

152 g (170 mL) of 0.5% Pd on $NaMgF_3$ prepared as described in Example 1 was charged to the reactor and reduced with hydrogen as indicated in Example 1. Hydrogen and chlorotrifluoroethylene (fluorocarbon 1113) were then passed into the reactor at an equimolar ratio with flow rates adjusted to give contact time varying from 2 seconds (463 g/h of fluorocarbon 1113) to 5 seconds (161 g/h of fluorocarbon 1113). During the first 22 hours of operation the furnace was kept at 200°C except for a 5.5 h period as indicated in Table 1. The contact times, % molar conversion of fluorocarbon 1113% molar yield of product fluorocarbon 1123 (based on 1113 converted) and % molar yield of by-product fluoro carbon 143 (based on 1113 converted) are summarized in Table 1. Each line represents two or more readings over the indicated period with percent conversion and percent yields rounded off to the nearest whole percent.

TABLE 1

| Time from start | Contact time | $H_2$: 113 mole ratio | % 1113 conversion | % 1123 yield | % 143 yield |
|---|---|---|---|---|---|
| 1—2.5 | 2.75 | 1 | 83—84 | 73—84 | 15—24 |
| 5—9 | 2 | 1 | 34—68 | 89—90 | 9 |
| 10—11 | 5 | 1 | 62—67 | 83—84 | 14 |
| 12 3/4—15 3/4 | 2 | 1 | 30—31 | 89—90 | 9—10 |
| 16.5—19 | 5 | 1 | 54—55 | 86—88 | 10—11 |
| 20—22 | 2 | 1 | 24—32 | 91 | 7—8 |
| 23.5—25 | 5 | 1 | 41—47 | 84—88 | 8—10 |
| 25.5—29 | 3 | 0.5 | 22—30 | 90—91 | 7—8 |
| 30—33 | 5 | 0.5 | 26—45 | 87—90 | 8—9 |
| 34—37 | 3 | 1 | 33—40 | 89—91 | 8—10 |
| 39—40.5 | 5 | 1 | 48—52 | 86—87 | 11—12 |
| 43—44 | 3 | 1 | 31—32 | 87—88 | 9—11 |
| 46—50* | 3 | 0.5 | 14—19 | 93 | 5—6 |
| 51—59 | 5 | 1 | 28—32 | 84—88 | 9—12 |

*Furnace at 250°C.

The catalyst was then regenerated by heating the furnace to 550°C, passing air through the reactor for about 3 h, reducing the furnace temperature to 400°C and flowing hydrogen through the reactor 7 h as the furnace temperature was gradually lowered to 200°C.

The catalytic dechlorination of fluorocarbon 1113 was then resumed as indicated in Table 2. The molar feed ratios of $H_2$ to fluorocarbon 1113 was varied between 1:1 and 0.5:1, the contact time was varied between 3 and 5 seconds, the furnace temperature was varied between 150°C and 200°C.

TABLE 2

| Time from start | Contact time | Temperature | H$_2$: 1113 mole ratio | % 1113 conversion | % 1123 yield | % 143 yield |
|---|---|---|---|---|---|---|
| 60.5 h | 5 s | 200 | 1 | 78 | 83 | 15 |
| 61.5—64 | 5 | 150 | 1 | 64—65 | 82—83 | 15—16 |
| 66—72.5 | 3 | 150 | 0.5 | 26—32 | 89—91 | 8—9 |
| 74—81 | 3 | 150 | 0.5 | 19—27 | 90—91 | 7—8 |
| 82.5—92 | 5 | 200* | 1 | 29—53 | 86—90 | 8—12 |
| 116—119 | —Activity check with fluorocarbon 113—low conversion; regeneration at 550°C with air | | | | | |
| 120—125 | 5 | 200 | 1 | 48—65 | 88—85 | 13—15 |
| | Catalyst used for unrelated fluorocarbon studies; regeneration at 550°C; catalyst then idle for 1 week | | | | | |
| 136—140 | 5 | 200 | 1 | 66—82 | 79—85 | 14—20 |

*Furnace cooled down for a few hours causing temporary drop in reactor.

Example 3

Dechlorination of fluorocarbon 113 with NaMgF$_3$ supported catalyst

Using the catalyst of Example 2 after 92 hours and the reaction of Example 2, fluorocarbon 113 and hydrogen were passed over the catalyst at molar ratios of 2:1, 1.3:1 and 1:1, at furnace temperatures of 200°C, 300°C and 500°C and at contact times at 3, 4.2 and 5 seconds. The results, as indicated in Table 3, are of enhanced, but still low conversions of fluorocarbon 113 after reactivation.

5

TABLE 3

| Time from start | Contact time | Temperature | H$_2$: 113 mole ratio | % 113 conversion | % 1123 yield | % 1113 yield | % 143 yield | % 133 yield | % 123a yield |
|---|---|---|---|---|---|---|---|---|---|
| 93 | 5 | 200 | 2:1 | 5 | 46 | 29 | — | 28 | — |
| 94 | 5 | 200 | 2:1 | 6 | 33 | 47 | — | 23 | — |
| 95 | 5 | 200 | 2:1 | 8 | 42 | 29 | — | 24 | — |
| 96 | 5 | 300 | 2:1 | 14 | 49 | 39 | — | 11 | — |
| | | | Regenerated at 550°C with air | | | | | | |
| 97 | 4.2 | 200 | 1.3:1 | 22 | 40 | 11 | 30 | 19 | — |
| 98 | 4.2 | 200 | 1.3:1 | 13 | 29 | 9 | 37 | 25 | — |
| 100 | 5 | 200 | 2:1 | 9 | 41 | 11 | 37 | 33 | — |
| 107 | 3 | 500 | 1:1 | 40 | 6 | 85 | — | 3 | — |
| 108 | 3 | 500 | 1:1 | 15 | — | 95 | — | 1 | — |
| 110 | 3 | 500 | 1:1 | 15 | — | 82 | — | 4 | 14 |

6

0 053 6570 053 657

## 0 053 657

Example 4

Using the catalyst and reactor of Example 2, fluorocarbon 113, fluorocarbon 1113 and hydrogen were passed over the catalyst at a molar ratio of 1:1:2 with a 200°C furnace temperature and a 5 second contact time. The results are displayed in Table 4 with the % 1113 conversion based on the net decrease in 1113 observed. Yields are based on fluorocarbons 1113 and 113 converted.

### TABLE 4

| Time from start | % 113 conversion | % 1113 conversion | % 1123 yield | % 143 yield | % 133 yield | % 123a yield |
|---|---|---|---|---|---|---|
| 127 | 13 | 44 | 82 | 16 | 4 | 0.7 |
| 128 | 13 | 37 | 81 | 14 | 4 | 1.6 |
| 129 | 12 | 35 | 81 | 15 | 4 | 0.8 |
| 130 | 30 | 28 | 81 | 14 | 3 | 0.7 |
| 131 | 30 | 24 | 81 | 14 | 3 | 0.6 |

Examples 5—7

Similar reactions with the sodium magnesium fluoride alone as catalyst or nickel on sodium magnesium fluoride were effective to convert fluorocarbon 113 to fluorocarbon 1113 at elevated temperatures above 400°C so long as hydrogen levels were kept low as described below. Whenever sufficient hydrogen was provided to produce fluorocarbon 1123, the high temperature apparently caused decomposition of fluorocarbon 1123, apparently to a polymer.

Similar reactions with palladium on carbon produce fluorocarbons 1113 and 1123 as described in the prior art but, eventually, the catalyst is deactivated by carbonaceous or polymer deposits. Unlike the catalysts used in the present invention, carbon-based catalysts cannot be regenerated with air.

Example 5

Dechlorination of fluorocarbon 113 with uncoated $NaMgF_3$

Using freshly activated sodium magnesium fluoride catalyst prepared as in Example 1 but without coating and the reactor arrangement described in Example 2, fluorocarbon 113 was reacted with hydrogen at 500°C. Conversions and yield data are shown in Table 5. Similar results are obtained with uncoated potassium magnesium fluoride. These results show lower fluorocarbon 113 conversions versus similar runs when copper, iron or nickel coated $NaMgF_3$ was used. No deactivation of the uncoated $NaMgF_3$ was observed at 500°C during the first 108 hours of reaction. After 108 hours, variable studies at 535°C showed initial higher activity but catalyst activity decline in about 20 hours. Regeneration with oxygen at 550°C to maintain the higher activity at 535°C was required. Periodic catalyst activity checks were made at 500°C by repeating a standard run. After 330 hours, the catalyst activity was equal to that observed at 108 hours. The standard run data are shown in Table 5.

7

TABLE 5

| Time from start | Contact time | Temperature °C | $H_2$: 113 mole ratio | % 113 conversion | % 1123 yield | % 1113 yield | % 133 yield | % 123a yield |
|---|---|---|---|---|---|---|---|---|
| 88h | 3 s | 500 | 0.5 | 21.3 | 7.12 | 83.90 | 5.66 | 3.56 |
| 108 | 3 | 500 | 0.5 | 25.5 | 8.06 | 84.52 | 3.49 | 3.92 |
| 130 | 3 | 500 | 0.25 | 9.9 | 5.18 | 89.83 | 2.54 | 2.44 |
| Variable Study—$O_2$ Regeneration 550°C | | | | | | | | |
| 218 | 3 | 500 | 0.5 | 26.5 | 7.79 | 82.18 | 3.72 | 6.31 |
| Variable Study—$O_2$ Regeneration 550°C | | | | | | | | |
| 330 | 3 | 500 | 0.5 | 25.1 | 6.00 | 84.46 | 2.84 | 6.70 |

Example 6

Dechlorination of fluorocarbon 113 with nickel on NaMgF$_3$

NaMgF$_3$ catalyst was prepared as in Example 1, but NiCl$_2$ was applied instead of PdCl$_2$. The concentration of nickel on the catalyst was 8.3%. Results are summarized in Table 6. The catalyst was more active but required more frequent regeneration. Catalyst regeneration by burning off a carbon deposit was required about ever 36 hours. No loss in catalyst activity was observed after regeneration.

TABLE 6

| Time from start | Contact time | Temp °C | H$_2$: 113 mole ratio | Mole % 113 conversion | Mole % 1123 yield | Mole % 1113 yield | Mole % 133 yield | Mole % 123a yield |
|---|---|---|---|---|---|---|---|---|
| 0—18h | 3.04s | 400 | 1.0 | 15.2 | 5.50 | 87.25 | 2.44 | 4.82 |
| 52—68 | 3.09 | 400 | 1.0 | 16.2 | 3.30 | 88.21 | 4.72 | 4.95 |
| 154—172 | 3.18 | 400 | 1.0 | 14.8 | 3.89 | 87.92 | 3.47 | 4.72 |
| 70—86 | 3.06 | 425 | 1.0 | 13.7 | 7.10 | 86.83 | 2.26 | 3.80 |
| 100—120 | 3.06 | 450 | 1.0 | 21.7 | 4.68 | 89.37 | 2.03 | 3.92 |
| 134—144 | 3.10 | 450 | 0.99 | 19.7 | 4.38 | 89.21 | 2.44 | 3.98 |
| 36—50 | 3.10 | 500 | 0.99 | 71.4 | 5.68 | 78.13 | 3.55 | 10.20 |
| 264—284 | 3.09 | 500 | 1.0 | 49.6 | 99.6 | 78.79 | 3.68 | 7.57 |

Example 7

Dechlorination of fluorocarbon 113 with copper on NaMgF$_3$

A NaMgF$_3$ catalyst was prepared as in Example 1, but CuCl$_2$ was applied instead of PdCl$_2$. The concentration of copper on the catalyst was 6.3%. Results are summarized in Table 7.

TABLE 7

| Contact time | Temp °C | H$_2$: 113 mole ratio | % 113 conversion | Mole % 1123 yield | Mole % 1113 yield | Mole % 133 yield | Mole % 123a yield |
|---|---|---|---|---|---|---|---|
| 3.24 s | 450 | 0.99 | 12.9 | 1.5 | 92.4 | 2.2 | 3.9 |
| 6.34 | 450 | 0.99 | 18.8 | 5.7 | 86.5 | 2.5 | 5.3 |
| 6.19 | 475 | 0.99 | 35.6 | 6.4 | 85.2 | 2.4 | 6.0 |
| 3.26 | 475 | 0.20 | 16.9 | 0 | 96.0 | 0.4 | 3.6 |
| 3.23 | 475 | 0.97 | 23.8 | 6.2 | 87.0 | 2.2 | 4.6 |
| 3.39 | 500 | 0.20 | 20.1 | 0 | 93.6 | 0 | 6.4 |
| 3.27 | 500 | 0.97 | 38.9 | 6.5 | 86.2 | 2.4 | 4.8 |

**Claims**

1. A process for producing chlorotrifluoroethylene or trifluoroethylene by the dechlorination in the vapor phase of 1,1,2-trichloro-1,2,2-trifluoroethane or chlorotrifluoroethylene with hydrogen in the presence of a supported platinum-group metal catalyst, wherein a feed of 1,1,2-trichloro-1,2,2-trifluoroethane, chlorotrifluoroethylene or mixtures thereof are reacted with hydrogen at a reaction temperature of 175 to 300°C in the presence of a supported platinum-group metal catalyst, characterized in that the catalyst support is selected from the group consisting of sodium magnesium fluoride and potassium magnesium fluoride, the catalyst is contacted with an oxygen-containing gas at a temperature between 400 and 600°C to reactivate the catalyst, and the feed is reacted with hydrogen in the presence of the reactivated catalyst at the reaction temperature.

2. The process of claim 1 wherein the feed is a mixture of 1,1,2-trichloro-1,2,2-trifluoroethane and chlorotrifluoroethylene, with trifluoroethylene being recovered as product.

3. The process of claim 1 wherein the reaction temperature is between 200°C and 250°C.

4. The process of claim 1 wherein the catalyst is palladium.

5. A process for producing chlorotrifluoroethylene by the catalytic dechlorination in the vapor phase of 1,1,2-trichloro-1,2,2-trifluoroethane feed with hydrogen at a temperature of between 400°C and 600°C in the presence of magnesium fluoride as a catalyst or as a catalyst support coated with at least one of iron, copper and nickel, characterized in that the uncoated catalyst or the coated catalyst support is selected from sodium magnesium fluoride and potassium magnesium fluoride, and the catalyst periodically reactivated by contacting the catalyst with an oxygen-containing gas at a temperature between 400°C and 600°C.

6. The process of claim 5 wherein the catalyst is sodium magnesium fluoride.

7. The process of claim 6 wherein the catalyst is sodium magnesium fluoride coated with nickel.

8. The process of claim 7 wherein the reaction temperature is between 475°C und 550°C.

**Patentansprüche**

1. Verfahren zum Erzeugen von Chlortrifluorethylen oder Trifluorethylen durch Dechlorieren von 1,1,2-Trichlor-1,2,2-trifluorethan oder Chlortrifluorethylen mit Wasserstoff in der Dampfphase in Gegenwart eines auf einem Träger befindlichen Katalysators aus einem Platinmetall, in dem ein aus 1,1,2 - Trichlor - 1,2,2 - trifluorethan, Chlortrifluorethylen oder einem Gemisch derselben bestehendes Einsatzprodukt bei Reaktionstemperaturen von 175 bis 300°C mit Wasserstoff in Gegenwart eines auf einem Träger befindlichen Katalysators aus einem Platinmetall umgesetzt wird, dadurch gekennzeichnet, daß der Katalysatorträger ausgewählt ist aus der Klasse, die aus Natriummagnesiumfluorid und Kaliummagnesiumfluorid besteht, daß der Katalysator zu seiner Reaktivierung mit einem sauerstoffhaltigen Gas bei einer Temperatur zwischen 400 und 600°C in Berührung gebracht wird, und daß das Einsatzgut mit Wasserstoff in Gegenwart des reaktkvierten Katalysators bei der Reaktionstemperatur umgesetzt wird.

2. Verfahren nach Anspruch 1, dadurch gekennzeichnet, daß das Einsatzprodukt ein Gemisch von 1,1,2 - Trichlor - 1,2,2 - trifluorethan und Chlortrifluorethylen ist und Trifluorethylen als Produkt gewonnen wird.

3. Verfahren nach Anspruch 1, dadurch gekennzeichnet, daß die Reaktionstemperatur zwische 200 und 250°C beträgt.

4. Verfahren nach Anspruch 1, dadurch gekennzeichnet, daß der Katalysator Palladium sit.

5. Verfahren zum Erzeugen von Chlortrifluorethylen durch die katalytische Dechlorierung von 1,1,2-Trichlor-1,2,2-trifluorethan als Einsatzprodukt mit Wasserstoff bei einer Temperatur zwischen 400 und 600°C in Gegenwart von Magnesiumfluorid als Katalysator oder als mit mindestens einer der Substanzen Eisen, Kupfer und Nickel überzogenem Katalysatorträger, dadurch gekennzeichnet, daß der nicht-überzogene Katalysator oder der überzogene Katalysatorträger aus Natriummagnesiumfluorid oder Kaliummagnesiumfluorid besteht und daß der Katalysator dadurch periodisch reaktiviert wird, daß der Katalysator mit einem sauerstoffhaltigen Gas bei einer Temperatur zwischen 400 und 600°C in Berührung gebracht wird.

6. Verfahren nach Anspruch 5, dadurch gekennzeichnet, daß der Katalysator Natriummagnesiumfluorid ist.

7. Verfahren nach Anspruch 6, dadurch gekennzeichnet, daß der Katalysator nickelüberzogenes Natriummagnesiumfluorid ist.

8. Verfahren nach Anspruch 7, dadurch gekennzeichnet, daß die Reaktionstemperatur zwischen 475 und 550°C beträgt.

**Revendications**

1. Procédé de production de chlorotrifluoroéthylène ou de trifluoroéthylène par déchloruration en phase vapeur de 1,1,2 - trichloro - 1,2,2 trifluoroéthane ou de chlorotrifluoroéthylène en présence d'hydrogène et d'un catalyseur métallique du groupe du platine disposé sur un support dans lequel on feit réagir un courant d'alimentation de 1,1,2 - trichloro - 1,2,2 - trifluoroéthane, chlorotrifluoroéthylène ou de mélanges de ces derniers avec de l'hydrogène à une température de réaction de 175 à 300°C en présence d'un catalyseur métallique du groupe du platine disposé sur un support caractérisé en ce que le support du catalyseur est du fluorure de sodium magnésium ou du fluorure de potassium magnésium, le catalyseur étant mis en contact avec un gaz contenant de l'oxygène à une température comprise entre 400 et 600°C pour réactiver le catalyseur, et le courant précité réagit avec l'hydrogène en présence du catalyseur réactivé à la température.

2. Procédé selon la revendication 1 dans lequel le courant consiste en un mélange de 1,2,2 - trichloro - 1,2,2 - trifluoroéthane et chlorotrifluoroéthylène, le trifluoroéthylène étant récupéré en tant que produit.

3. Procédé selon la revendication 1, dans lequel la température de réaction est comprise entre 200 et 250°C.

4. Procédé selon la revendication 1 dans lequel le catalyseur est du palladium.

5. Procédé pour la production de chlorotrifluoroéthylène par déchloruration catalytique en phase vapeur d'un courant de 1,1,2 - trichloro - 1,2,2 - trifluoroéthane et d'hydrogène à une température comprise entre 400 et 600°C en présence de fluorure de magnésium en tant que catalyseur ou en tant que

**0 053 657**

support de catalyseur revêtu d'au moins un (des métaux) fer, cuivre ou nickel caractérisé en ce que le catalyseur non revêtu ou revêtu est du fluorure de potassium magnésium et le catalyseur est réactivé périodiquement par mise en contact dudit catalyseur avec un gaz contenant de l'oxygène à une température comprise entre 400 et 600°C.

6. Procédé selon la revendication 5 dans lequel le catalyseur est du fluorure de sodium magnésium.

7. Procédé selon la revendication 6 dans lequel le catalyseur est du fluorure de sodium magnésium revêtu de nickel.

8. Procédé selon la revendication 7 dans lequel la température de réaction est comprise entre 475 et 550°C.